# EUROPEAN PATENT SPECIFICATION

(11) **EP 4 479 378 B1**
(45) Date of publication and mention of the grant of the patent: **19.08.2026**
(21) Application number: 23704195.9
(22) Date of filing: 15.02.2023
(51) Int. Cl.: C07C 303/16, C07C 309/04

(54) **PROCESS FOR PRODUCING ALKYL SULFONIC ACID**
VERFAHREN ZUR HERSTELLUNG VON ALKYLSULFONSÄURE
PROCÉDÉ DE PRODUCTION D'ACIDE ALKYLSULFONIQUE

(30) Priority: 18.02.2022 EP 22157498
(43) Date of publication of application: 25.12.2024
(73) Proprietor: BASF SE, 67056 Ludwigshafen am Rhein (DE)
(72) Inventor: ZEPP, Brigitte, 67056 Ludwigshafen (DE); DIENES, Christian, 67056 Ludwigshafen (DE); BORGMEIER, Frieder, 67056 Ludwigshafen (DE); REIFEGERSTE, Simon, 67056 Ludwigshafen (DE); SPIELMANN, Jan, 67056 Ludwigshafen (DE)
(74) Representative: BASF IP Association
(86) International application number: PCT/EP2023/053713
(87) International publication number: WO 2023/156430

(56) References cited:
- EP-B1- 1 133 470
- US-B1- 6 531 629

## Description

The invention relates to a process for producing alkyl sulfonic acid in a reactor which contains a liquid phase comprising aqueous nitric acid, wherein dialkyl disulfide is fed into the liquid phase in the reactor and a crude reaction product is formed in the reactor by chemical reaction of the dialkyl disulfide with the nitric acid.

Alkyl sulfonic acids, in particular methanesulfonic acid (MSA), are used in a large number of applications in pure form and in admixture with water or other solvents. The use of MSA is particularly widespread in electroplating, tinplate production and wire tinning. Alkyl sulfonic acids are also employed as solvent or as catalyst in alkylation and esterification reactions for example. A further field of application for alkyl sulfonic acids is the production of biodiesel where the typically employed sulfuric acid may be replaced by alkyl sulfonic acids on account of the improved performance properties of the latter.

Alkyl sulfonic acids are also an alternative to phosphoric-acid-containing cleaning product formulations. Since MSA in particular forms readily soluble salts and is readily biodegradable, the alternative use of alkyl sulfonic acid can play a role in water pollution control. A further use of alkyl sulfonic acids is the catalytic alkylation of benzene or substituted benzene with olefins to produce linear alkylbenzene. This in turn is a starting material for the production of linear alkylbenzenesulfonic acids, the most commonly used surfactants.

For producing alkyl sulfonic acid, different processes are known. According to the descriptions of US 4,052,445 and US 4,239,696 dialkyl disulfide reacts with hydrogen peroxide. In US 4,052,445 the reaction is carried out in an inert medium in the presence of a catalyst. The process of US 4,239,696 uses an alkyl sulfonic acid as liquid medium. A further process for producing alkyl sulfonic acids by oxidation of a dialkyl disulfide with hydrogen peroxide is described in EP-A 0 313 939. Here, the dialkyl disulfide is mixed with aqueous hydrochloric acid.

A process for producing alkyl sulfonic acids by oxidation of a sulfur containing starting compound, for example dialkyl disulfide, with an oxygen containing fluid like air, oxygen enriched air or pure oxygen is described in EP-A 3 453 447. The reaction is carried out in a reaction vessel with an expansion vessel on its top. The reaction vessel preferably is a stirred tank reactor.

WO-A 2015/086645 and WO-A 2015/085522 both disclose processes for producing alkyl sulfonic acids by oxidizing a dialkyl disulfide in solution in an alkyl sulfonic acid in the presence of catalytic amounts of nitric acid.

Besides these processes which use oxygen or hydrogen peroxide as oxidizing agent, further processes are known in which the dialkyl disulfide reacts with nitric acid forming alkyl sulfonic acid, a mixture of gaseous nitrogen oxides like nitrogen dioxide, nitrogen monoxide, and water. The molar ratio of nitrogen dioxide and nitrogen monoxide that is formed in the process depends on the molar ratio of dialkyl disulfide to nitric acid that is used as can be seen in reaction equations (1) and (2).

3 R-S-S-R + 10 HNO₃ → 6 RSO₃H + 10 NO + 2 H₂O (1)

R-S-S-R + 10 HNO₃ → 2 RSO₃H + 10 NO₂ + 4 H₂O (2)

Such a process is described for example in US-B 6,531,629. In this case, the dialkyl disulfide is fed into a liquid phase containing the nitric acid. As mixtures of dialkyl disulfides with nitrogen oxides may be explosive, the dialkyl disulfide is added to the reaction mixture immersed under the surface of the liquid. Several moles of gaseous nitrogen oxides are formed in the reaction per each mole of dialkyl disulfide. The evolution of these large amounts of gases induces a strong flow in the reaction mixture and results in vigorous mixing of the liquid contents. By this process, the conversion and selectivity compared to other processes can be increased. Nonetheless, there still are byproducts formed like sulfuric acid.

Sulfuric acid impacts the properties of the alkyl sulfonic acids and especially methanesulfonic acid for example in electroplating applications and, thus, needs to be removed from the product stream by a separation process. The stream leaving the process ("purge" or "purge stream") is proportional in size to the amount of sulfuric acid generated in the reaction. Therefore, even if only a slight improvement in selectivity of the reaction is achieved, the overall yield of the process can be significantly improved.

For efficient separation, the sulfuric acid cannot easily be concentrated to any desired value. This leads to purge streams much larger than the actual amount of sulfuric acid generated, causing losses of the desired product.

Therefore, it was an object of the present invention to improve the process for producing alkyl sulfonic acid by reaction of dialkyl disulfide with nitric acid, where the formation of by-products is reduced.

This object is achieved by a process for producing alkyl sulfonic acid in a reaction apparatus which contains a liquid phase comprising aqueous nitric acid, wherein dialkyl disulfide is fed into the liquid phase in the reaction apparatus and a crude reaction product is formed in the reaction apparatus by chemical reaction of the dialkyl disulfide with the nitric acid, wherein feeding the dialkyl disulfide comprises at least one of:
- the liquid dialkyl disulfide is fed into the reaction apparatus through an orifice having a hydraulic diameter of less than 5 mm;
- the liquid dialkyl disulfide is fed into the reaction apparatus with an inlet velocity of at least 0.6 m/s.

The term "orifice" here refers to any opening in the reaction setup through which liquid dialkyl disulfide may be fed into the liquid phase in the reaction apparatus. Orifices may for example be openings on lines or at the end of lines and nozzles or feeding points of dialkyl disulfide into lines containing nitric acid or the liquid phase from the reactor.

The hydraulic diameter is defined as the diameter of a circular area which has the same cross-sectional area as the described orifice and can be calculated by ${d}_{h} = \frac{4 A}{U}$ with the hydraulic diameter dₕ, the cross-sectional area of the orifice A, and the perimeter of the orifice U.

The crude reaction product obtained in the reaction usually is fed into a purifying process to obtain pure alkyl sulfonic acid. In the purifying process, by-products and reactants which did not react, usually are removed by distillation, wherein distillation preferably is carried out in at least two distillation stages. If the alkyl sulfonic acid is methanesulfonic acid or ethyl sulfonic acid, the sulfuric acid is a high boiler compared to the alkyl sulfonic acid and for this reason accumulates in the bottom of each distillation column. As sulfuric acid is detrimental to product quality and the sulfuric acid concentration in the pure alkyl sulfonic acid preferably is below 50 ppm, it is necessary to remove the sulfuric acid by a purge stream. Due to the technical limitations of the separation process, the purge stream which is withdrawn at the bottom of the distillation column still contains a high concentration of the alkyl sulfonic acid. For reducing the amount of alkyl sulfonic acid being withdrawn from the process and thus increasing the yield of alkyl sulfonic acid, it is necessary to minimize the purge stream. However, if the amount of sulfuric acid produced as by-product in the reaction is reduced, the purge stream can be reduced, too.

Surprisingly it has been shown that by using an orifice having a hydraulic diameter of less than 5 mm, more preferred of less than 3 mm and particularly of less than 2 mm, by feeding the liquid dialkyl disulfide into the reactor with an inlet velocity of at least 0.6 m/s, preferably of at least 1.8 m/s, particularly preferably at least 2.5 m/s, the formation of by-products produced in the reaction of dialkyl disulfide with nitric acid for producing alkyl sulfonic acid, particularly the formation of sulfuric acid, can be reduced. By additionally and further preferably feeding the dialkyl disulfide into the reactor through a plurality of orifices, wherein the number of orifices is larger than 1.0 orifices per cubic meter reaction volume, more preferred larger than 1.2 orifices per cubic meter reaction volume and particularly of larger than 1.7 orifices per cubic meter reaction volume, a further reduction of by-products may be possible. "Reaction volume" in this context refers to the volume of liquid reactants present in the reactor.

Further, by the reduction of the by-products formed in the reaction, like sulfuric acid, the purification process of the alkyl sulfonic acid can be facilitated, too, and simultaneously, the losses of product also are reduced.

The alkyl sulfonic acid preferably is methanesulfonic acid or ethyl sulfonic acid. If the alkyl sulfonic acid is methanesulfonic acid, the dialkyl disulfide used in the production process is dimethyl disulfide. Correspondingly, if the alkyl sulfonic acid is ethyl sulfonic acid, the dialkyl disulfide is diethyl disulfide. Particularly preferably, the alkyl sulfonic acid is methanesulfonic acid and the dialkyl disulfide is dimethyl disulfide.

The reaction usually is carried out at a temperature in the range between 50 °C to 150 °C, preferably at a temperature in the range from 80 °C to 140 °C and at an operating pressure in the range from 500 mbar to 8 bar, preferably at atmospheric pressure.

The reaction apparatus, in which the reaction is carried out, may comprise one reactor or a battery of at least two reactors. Preferably, for increasing the yield, the reaction is carried out in two reactors. In this case, the liquid dialkyl disulfide and the nitric acid are fed into a first reactor, in which a first crude reaction product is formed, the first crude reaction product is fed into a second reactor, in which the reaction for forming the alkyl sulfonic acid is completed, thereby forming the crude reaction product. The reactor, or, if a battery of at least two reactors is used, the reactors, can be reactors with a high degree of back-mixing like stirred tank reactors or loop reactors. Alternatively, the reactor or the reactors may be reactors with a low degree of back-mixing like a tubular flow reactor. Preferably, the reactor or the reactors is/are reactor(s) with a high degree of back-mixing. If more than one reactor is used for carrying out the reaction, it is possible that all reactors have the same construction, e.g. all reactors are stirred tank reactors or loop reactors, or that all reactors are different, for example one stirred tank reactor and one loop reactor.

The reactor, if the reaction is carried out in only one reactor, or at least one of the reactors into which dialkyl disulfide is fed, if the reaction is carried out in a battery of at least two reactors, may be a reactor which comprises an external fluid circulation. In a reactor which comprises an external fluid circulation, the dialkyl disulfide may be fed either directly into the liquid phase in the reactor, for example via an orifice, or as an alternative into the external fluid circulation. For feeding the dialkyl disulfide each of the feeding alternatives, namely feeding the dialkyl disulfide into the reactor through an orifice having a hydraulic diameter of less than 5 mm, or feeding the liquid dialkyl disulfide into the reactor with an inlet velocity of at least 0.6 m/s, and additionally feeding the liquid dialkyl disulfide into the reactor through a plurality of orifices, wherein the number of orifices is larger than 1.0 orifices per cubic meter reaction volume, can be used. If the dialkyl disulfide is fed into the external fluid circulation of the reactor, it is fed through at least one orifice. Depending on the diameter of the external fluid circulation line and if the dialkyl disulfide is fed through a plurality of orifices, it is particularly preferred if the line through which the dialkyl disulfide is fed ends in the external fluid circulation in the form of a ring or as a sphere or a distributor head like a shower head in which the orifices are formed. If the dialkyl disulfide is fed into the external fluid circulation, particularly preferably a mixing nozzle is used for feeding the dialkyl disulfide.

Alternatively, the reactor, if the reaction is carried out in only one reactor, or at least one reactor into which dialkyl disulfide is fed, if the reaction is carried out in a battery of at least two reactors, may be a jet loop reactor. A jet loop reactor usually comprises a vessel with an inner tube having open ends, the inner tube being arranged concentrically in the vessel. For introducing a component like dialkyl disulfide into the liquid phase, usually a nozzle is used which is arranged at one open end of the inner tube to introduce the liquid phase into the inner tube. By introducing the liquid phase into the inner tube, a flow is generated in the liquid phase inside the jet loop reactor from the nozzle through the inner tube, around the end of the inner tube which is opposite the nozzle and back in the space between the outside of the inner tube and the walls of the vessel. The nozzle thereby may be arranged at any end of the inner tube. If the nozzle is arranged at the upper end of the inner tube, the liquid flows downwards in the inner tube and upwards in the space between the inner tube and the walls of the vessel and if the nozzle is arranged at the lower end of the inner tube, the liquid flows in the opposite direction in the jet loop reactor. Further, it is also possible to arrange the nozzle at any position inside the inner tube. In this case the flow direction of the liquid in the jet loop reactor depends on the nozzle orientation and, thus, on the direction in which the component leaves the nozzle. It is particularly preferred to arrange the nozzle inside the inner tube submerged below the surface of the liquid phase to prevent the formation of explosive gaseous atmospheres inside the reactor.

A jet loop reactor can also be constructed without an inner tube. In this case the orientation of the nozzle needs to be in a way that the introduced liquid phase from the nozzle flows downward into the vessel. If no inner tube is used, it is particularly preferred to arrange the nozzle below the surface of the liquid phase to prevent the formation of explosive gaseous atmospheres inside the reactor.

If a jet loop reactor is used for the inventive process, the dialkyl disulfide is fed into the jet loop reactor either into the recirculation line or directly into the nozzle with an inlet velocity of at least 0.6 m/s, preferably of at least 1.7 m/s, even more preferred 2.5 m/s.

Further it is possible to feed the dialkyl disulfide into the reactor into the recirculation line or into the nozzle with an inlet velocity of at least 0.6 m/s and additionally through a plurality of orifices, wherein the plurality of orifices preferably is arranged in a ring which surrounds the nozzle concentrically.

If the dialkyl disulfide is fed into the jet loop reactor only via a plurality of orifices, it is preferred to arrange the orifices either in a ring or at least two concentric rings or in a distributor head which are positioned underneath the surface level of the liquid.

As a further alternative and preferably, the reactor, if the reaction is carried out in only one reactor, or at least one reactor into which dialkyl disulfide is fed, if the reaction is carried out in a battery of at least two reactors, particularly the first reactor of the battery of at least two reactors, is a tank reactor. The tank reactor may be a stirred tank reactor or a tank reactor without an additional mixing device. If the tank reactor does not comprise an additional mixing device, the liquid in the tank reactor is mixed by introducing the dialkyl disulfide into the tank reactor by one of the inventive alternatives. Independently of whether a tank reactor without mixing device or a stirred tank reactor are used, each of the alternatives for feeding the dialkyl disulfide can be used. The dialkyl disulfide for example can be fed into the tank reactor through a plurality of nozzles which are arranged at the bottom and/or the walls of the reactor, through a dip tube having at least one opening or through a distributor, particularly a distributor ring, positioned in the liquid having a plurality of orifices. If the dialkyl disulfide is fed into the reactor via a distributor, the line to which the distributor is connected may be introduced into the reactor at any position, for example from the bottom or, from the top, which means that the distributor forms the end of a dip tube. Preferably, the line to which the distributor is connected is introduced into the reactor from the side in a way that the line is below the surface of the liquid.

Independently of the type of the reactor, it is necessary that the dialkyl disulfide is fed into the liquid phase in the reactor to prevent formation of explosive mixtures of nitrogen oxides and dialkyl disulfides. During the reaction of dialkyl disulfide and nitric acid in the liquid phase large amounts of gaseous nitrogen oxides are formed and the gas flow causes vigorous mixing of the liquid contents.

Further, independent of the type of reactor, the reactor may comprise an additional mixing device. The additional mixing device may be any mixing device known to a skilled person, for example a stirrer. Stirrers that can be used as mixing device preferably are high speed stirrers suitable for dispersing. Such stirrers for example are propeller stirrers, oblique blade stirrers, multistage impulse and countercurrent (MIG) stirrers, interference multistage impulse and countercurrent (interMIG) stirrers, or disk stirrers. The design of the stirrers may be single or multistage. When using a stirrer, it is further preferred if the vessel comprises baffles.

If the reaction is carried out in two reactors, the first reactor preferably is operated at a temperature in the range from 50 to 150 °C, particularly at a temperature in the range from 80 to 120 °C, and the second reactor into which the crude reaction product is transferred, preferably is operated at a temperature in the range from 100 to 150 °C, particularly at a temperature in the range from 130 to 150 °C.

The residence time in each reactor can be in the range from 10 minutes to 10 hours, preferably between 1 and 3 hours.

If the reaction is carried out in two reactors, usually, the dialkyl disulfide is largely oxidized in the first reactor, forming a first crude reaction product, which contains essentially the corresponding alkyl sulfonic acid and, in a small amount, incomplete oxidation products as well as excess nitric acid and small amounts of sulfuric acid. The yield of alkyl sulfonic acid in the first crude reaction product obtained in the first reactor is usually already greater than 60 %, preferably greater than 80 % or even greater than 90 %, based on the amount of dialkyl disulfide used. In the second reactor completion of the oxidation reaction takes place, as a result of which the yield of alkanesulfonic acid is usually increased to more than 90 %, preferably more than 93 % or even more preferred more than 95 %.

To obtain pure alkyl sulfonic acid, the crude reaction product which is obtained in the reactor or, if the reaction is completed in a battery of at least two reactors, particularly in two reactors, in the last reactor of the battery is worked-up. By working-up the crude reaction product, impurities like by-products or reactants which did not react, are removed. Working-up the crude reaction product preferably comprises at least one distillation step.

In the distillation step, high boilers and low boilers can be removed from the crude reaction product. In this context, "high boilers" mean components having a boiling temperature above the boiling temperature of the alkyl sulfonic acid and "low boilers" mean components having a boiling temperature below the boiling temperature of the alkyl sulfonic acid.

The main by-product which must be removed from the alkyl sulfonic acid is sulfuric acid and is concentrated in the distillation process. Besides the sulfuric acid, the concentrated sulfuric acid usually also contains alkyl sulfonic acid. To achieve a stationary process in which the sulfuric acid does not accumulate, it is necessary to remove a purge stream from that distillation column in which the sulfuric acid concentrates.

If the alkyl sulfonic acid is methane sulfonic acid or ethyl sulfonic acid, the sulfuric acid is a high boiler and, thus, concentrates in the bottom of the distillation. If, in this case, two distillation columns are used and in the first distillation column the low boilers are removed and in the second column the high boilers, the purge stream is withdrawn from the bottom of the second distillation column. If, on the other hand, the high boilers are removed in the first distillation column and a side stream or top stream from the first distillation column is transferred into the second distillation column in which particularly the low boilers are removed, the purge stream is withdrawn from the first distillation column. If only one distillation column is used, the pure methane sulfonic acid or ethane sulfonic acid usually is withdrawn as a side stream, the low boilers are withdrawn from the top of the distillation column and the high boilers including the sulfuric acid from the bottom. However, particularly preferably, if the alkyl sulfonic acid is methane sulfonic acid or ethyl sulfonic acid, working-up the alkyl sulfonic acid is carried out in two distillation columns by vacuum distillation as described in US-B 6,531,629, where in the first distillation column low boilers are removed and the bottom stream of the first distillation column is transferred into the second distillation column in which the pure alkyl sulfonic acid is obtained as a side stream, the top stream withdrawn from the second distillation column contains water, alkyl sulfonic acid, alkyl sulfonic alkyl ester and other low boilers and the bottom stream contains sulfuric acid, alkyl sulfonic acid and other high boilers.

Independently of the number of distillation columns used for working-up the crude alkyl sulfonic acid, the stream which comprises the sulfuric acid, also contains alkyl sulfonic acid. The amount of sulfuric acid in the crude product needs to be as low as possible, because the lower the amount of sulfuric acid, the smaller the purge stream can be at the same outlet concentration of sulfuric acid and, thus, the smaller is the amount of alkyl sulfonic acid withdrawn from the process in the purge stream. This reduction in the purge stream results in an increase in product yield. By the inventive process with the specific addition of the dialkyl disulfide, the amount of sulfuric acid in the process can be minimized and, thus, the amount of alkyl sulfonic acid withdrawn with the purge stream from the distillation also is minimized.

Illustrative embodiments of the invention are shown in the figures and explained in more detail in the following description.

In the figures:
- Figure 1: shows a schematic flow chart of a process for producing alkyl sulfonic acid;
- Figure 2: shows a tank reactor with orifices at the bottom and the walls of the reactor;
- Figure 3: shows a tank reactor with a distribution ring for feeding the dialkyl disulfide;
- Figure 4: shows a reactor with an external fluid circulation with supply for dialkyl disulfide in the reactor;
- Figure 5: shows a reactor with an external fluid circulation with supply for dialkyl disulfide in the external fluid circulation;
- Figure 6: shows a schematic flow chart of the experimental set-up

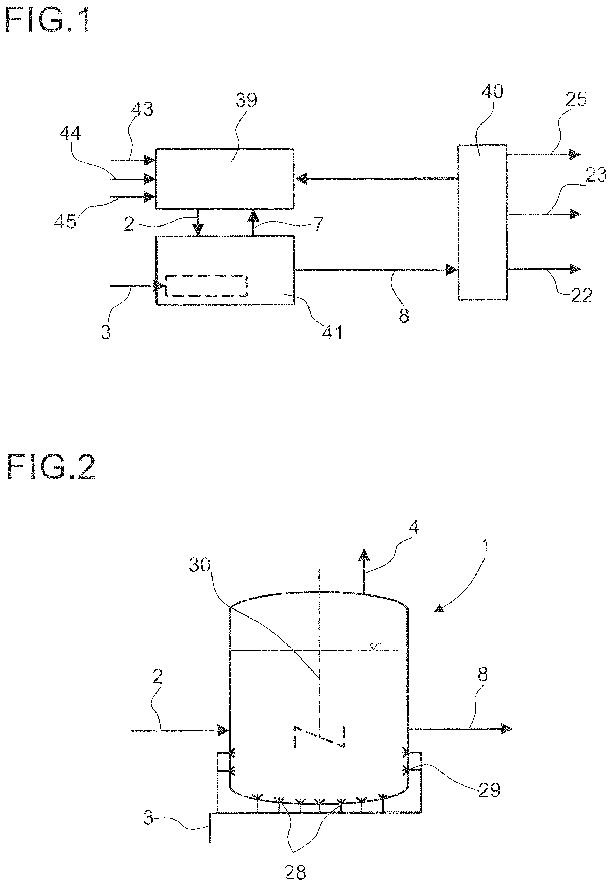

Figure 1 shows a schematic flow chart of a process for producing alkyl sulfonic acid.

For producing alkyl sulfonic acid, particularly methanesulfonic acid, nitric acid is fed into a reaction apparatus 41 via a first feed line 2 and dialkyl disulfide is fed into the reaction apparatus 41 via a second feed line 3.

The reaction apparatus 41 may comprise only one reactor or more than one reactor, for example two reactors.

The nitric acid fed into the reaction apparatus 41 preferably has a concentration from 20 to 100 % by weight, more preferred from 40 to 70 % by weight and particularly from 50 to 70 % by weight, and the dialkyl disulfide preferably is pure dialkyl disulfide having a purity of greater than 98 %. The molar ratio of dialkyl disulfide : nitric acid preferably is in a range from 1 : 2 to 1 : 20, more preferred in a range from 1 : 3 to 1 : 10 and particularly in a range from 1 : 3 to 1 : 6.

The reaction usually is carried out at a temperature in the range between 50 °C to 150 °C, preferably at a temperature in the range from 80 °C to 140 °C and at an operating pressure in the range from 500 mbar to 8 bar, preferably at atmospheric pressure. Gaseous components obtained in the reaction, which comprise nitrogen oxides like nitrogen monoxide and nitrogen dioxide, are transferred to a nitric acid regeneration system 39 via a gas line 7.

The liquid crude reaction product obtained in the reaction apparatus 41 is fed into a distillation 40 via a transfer line 8. In the distillation 40, the liquid crude reaction product is separated into a light fraction 25, a mid-boiler fraction 23, comprising the pure methanesulfonic acid and a bottom stream 22, which comprises high boilers.

The low boilers, which are obtained in the distillation 40 usually are condensed and the part which does not condense is withdrawn from the distillation 40 as light fraction 25. The condensed low boilers, which contain nitric acid, are partly transferred back into the distillation 40. That part of the condensed low boilers which is not transferred back into the distillation 40 is transferred to the nitric acid regeneration system 39.

The distillation 40 may comprise only one distillation column or, preferably, at least two distillation columns. If the distillation 40 comprises one distillation column, the light fraction 25 is withdrawn from the top of the column, the mid-boiler fraction 23 by a side take-off and the high boilers at the bottom of the column. If two distillation columns are used, in the first distillation column the high boilers and mid-boilers are separated and withdrawn at the bottom of the first distillation column and low boilers are withdrawn at the top of the first distillation column and transferred into the nitric acid regeneration system 39. The mid-boilers and the high boilers withdrawn at the bottom of the first distillation column are transferred into a second distillation column in which the high boilers are withdrawn at the bottom of the column as bottom stream 22, the mid-boiler fraction 23 comprising the pure alkane sulfonic acid is withdrawn as a side stream from the second distillation column and the light fraction 25 comprising remaining low-boilers is withdrawn at the top of the second distillation column.

For regenerating the nitric acid, besides the gaseous phase obtained in the reaction apparatus 41 and the condensed part of the low boilers of the distillation 40, air 43 and water 44 are fed into the nitric acid regeneration system 39. In the nitric acid regeneration system 39, the nitric acid transferred from the distillation 40 is concentrated and the nitrogen oxides, which are obtained in the reaction react with the water, thereby forming nitric acid. To compensate losses of nitric acid, fresh nitric acid can be added to the system via line 45.

Different embodiments of reactor configurations carrying out the inventive process are shown in figures 2 to 5. If the reaction apparatus 41 comprises a series of reactors for carrying out the inventive process, all the reactors may have a similar construction, but it is also possible to use different reactor designs for a series of reactors. For example in a series of two reactors, the first reactor may be a stirred tank reactor and the second reactor may be a tank reactor with an external fluid circulation line.

Figure 2 shows a tank reactor with orifices at the bottom and the walls of the reactor.

In the embodiment shown in figure 2, the reactor 1 is a tank reactor to which the first feed line 2 for feeding nitric acid is connected. For withdrawing the crude reaction product, the tank reactor comprises transfer line 8, and for removing evaporated low boilers and gaseous components, the gas line 4 is connected to the top of the tank reactor.

For feeding the dialkyl disulfide, the second feed line 3 is connected to orifices 28 at the bottom of the tank reactor and to orifices 29 at the walls of the tank reactor. The orifices 29 at the walls of the tank reactor are at such positions at the walls, that all orifices 29 at the walls of the tank reactor are placed below the liquid/gas phase boundary in the liquid phase. The orifices 28 at the bottom and orifices 29 at the walls of the reactor have a hydraulic diameter of less than 5 mm. Additionally, the amount of orifices 28 at the bottom and of orifices 29 at the walls is such that the number of orifices is larger than 1.0 orifices per cubic meter reaction volume.

The tank reactor may be an unstirred tank reactor or a stirred tank reactor. If the tank reactor is a stirred tank reactor, the tank reactor comprises a mixing device 30. As the mixing device 30 only is optional, it is shown in figure 2 with a dashed line. The mixing device 30 may be any type of stirrer known to the skilled person and as described above.

The crude reaction product is withdrawn from the reactor 1 via the transfer line 8 and gaseous components via a gas line 4.

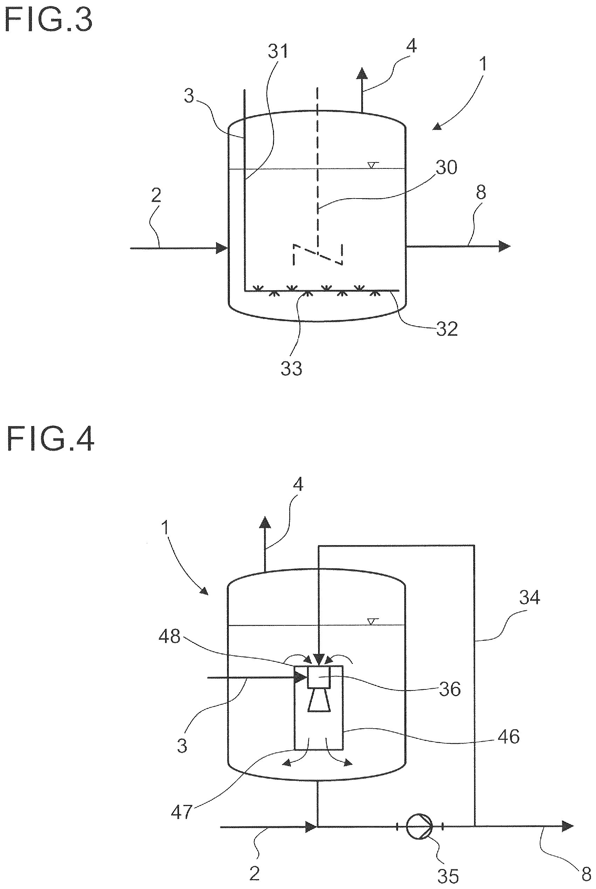

Figure 3 shows a tank reactor with a distribution ring for feeding the dialkyl disulfide.

The tank reactor, which is shown in figure 3, differs from the tank reactor shown in figure 2 in the feeding device for the dialkyl disulfide.

In contrast to the tank reactor shown in figure 2, the second feeding line 3 of the tank reactor of figure 3 is a dip tube 31 which is connected to a distributor 32. In the distributor 32, orifices 33 are formed for feeding the dialkyl disulfide. As the orifices 28, 29 in the bottom and wall of the tank reactor shown in figure 2, each orifice 33 has a hydraulic diameter of less than 5 mm and the number of orifices 33 in the distributor 32 is larger than 1.0 orifices per cubic meter reaction volume.

A further alternative for the reactor construction is shown in figure 4.

According to the embodiment shown in figure 4, the reactor 1 is a tank reactor with an external fluid circulation 34. In the reactor shown here, the first feed line 2 for feeding the nitric acid opens into the external fluid circulation 34. For circulating the liquid phase, a pump 35 is arranged in the external fluid circulation 34. The pump 35 may be any suitable pump for circulation of the liquid or gas liquid mixture, for example a centrifugal pump. The external fluid circulation 34 ends in an ejector nozzle 36 through which the liquid is injected into the tank reactor. The second feed line 3 for feeding the dialkyl disulfide ends in the ejector nozzle 36 and the dialkyl disulfide is sucked through the ejector nozzle into the liquid jet which leaves the nozzle. According to the invention, the liquid dialkyl disulfide is fed into the nozzle with an inlet velocity of at least 0.6 m/s. The mixture exiting the nozzle preferably is introduced into the tank contents below the liquid surface to prevent the formation of an explosive gas mixture. To generate a loop flow in the tank reactor, at least the nozzle outlet is surrounded by an inner tube 46. By injecting the liquid into the tank reactor via the ejector nozzle 36 the liquid flows through the inner tube 46 to a first end 47, surrounds the first end 47 of the inner tube 46 and flows outside of the inner tube 46 in the opposite direction to a second end 48 of the inner tube 46 and then into the inner tube 46 again. By this, the loop flow is generated in the reactor by which the components are mixed.

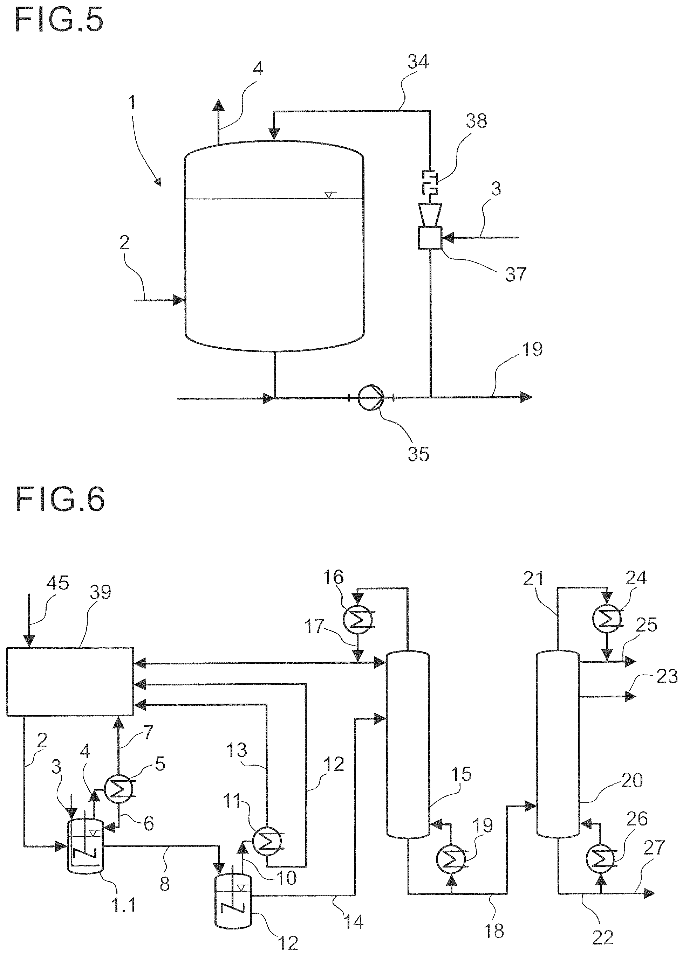

A reactor with an external fluid circulation with supply for dialkyl disulfide in the external fluid circulation is shown in figure 5.

As in the reactors shown in figures 2 and 3, the nitric acid is fed directly into the reactor via the first feed line 2.

In contrast to the reactor shown in figure 4, the second feed line 3 for feeding the dialkyl disulfide is connected to a nozzle 37 which is placed in the external fluid circulation 34. By using the nozzle 37, the dialkyl disulfide is fed into the external fluid circulation 34 such that the inlet velocity of the liquid jet leaving the nozzle 37 and, thus, also the inlet velocity of the dialkyl disulfide is at least 0.6 m/s.

To further mix the liquid in the external fluid circulation 34 and the dialkyl disulfide, it is preferred to provide a static mixer 38 in the external fluid circulation 34 downstream the nozzle 37.

### Examples

A setup according to figure 6 and to the following description was used for all examples given below. The inventive process was performed in the set up as follows, in which the reaction apparatus 41 comprises a first reactor 1.1 and a second reactor 1.2:
For producing alkyl sulfonic acid, particularly methanesulfonic acid, nitric acid is fed into a first reactor 1.1 via the first feed line 2 and dialkyl disulfide is fed into the first reactor 1.1 via the second feed line 3.

Due to the reaction conditions in the first reactor 1.1, at least some of the low boilers contained in the first reactor 1.1, for example nitric acid and water, evaporate and are withdrawn together with gaseous by-products from the first reactor 1.1 via a gas line 4 and fed into a first condenser 5. In the first condenser 5, condensable components condense and are fed back into the first reactor 1.1 via a recycle line 6. The non-condensed components, which comprise nitrogen oxides like nitrogen monoxide and nitrogen dioxide, are transferred to the nitric acid regeneration system 39 via a gas line 7. Preferably, the nitric acid obtained in the nitric acid regeneration system 39 is recycled into the process and fed into the first reactor 1.1 via the first feed line 2. Fresh nitric acid can be added via line 45 to compensate nitric acid losses.

From the first reactor 1.1 a stream is withdrawn via a transfer line 8 and fed into a second reactor 1.2.

As in the first reactor 1.1, gaseous by-products are formed and low boilers partially evaporate in the second reactor 1.2. The evaporated low boilers and gaseous by-products, like nitrogen oxides, are withdrawn via a gas line 10 and fed into the second condenser 11. In the second condenser 11 the condensable low boilers condense. The condensed low boilers, which contain nitric acid, are transferred to the regeneration for nitric acid via a feed line 12 and the non-condensed components comprising nitrogen oxides are transferred to the regeneration for nitric acid via a gas line 13.

The liquid crude reaction product obtained in the second reactor 1.2 is fed into a first distillation column 15 via a transfer line 14. In this first distillation column 15 light boilers are evaporated and condensed in a third condenser 16. The reflux 17 from the third condenser 16 is divided and transferred back to the head of column 15 or transferred to the nitric acid regeneration system 39. The concentrated bottom stream is heated in a first reboiler 19 and part of the bottom stream is transferred via a line 18 to a second distillation column 20. In the second distillation column 20, the stream from the line 18 is split into a top stream 21, a mid-boiler fraction 23, comprising the pure methanesulfonic acid and a bottom stream 22. The top stream 21 of the distillation column 20 is condensed in a fourth condenser 24 and part of the stream is withdrawn as the light fraction 25, whereas the rest of the condensed stream 21 is recirculated to the head section of the second distillation column 20. From the bottom stream 22 a purge stream 27 is branched off, before the stream is heated in a second reboiler 26 and recirculated to the bottom of the second distillation column 20.

### Example1:

The first reactor 1.1 was charged continuously, while continuously agitated, via the second feed line 3 with pure dimethyl disulfide (>98%) and with 55-65% strength nitric acid via feed line 2 in the DMDS (dimethyl disulfide): HNO3 molar ratio of 1 : 5,3.

Dimethyl disulfide is introduced submerged into the reaction mixture in the first reactor 1.1 via a distribution ring.

The distribution ring has 1.7 orifices per cubic meter of reaction volume in the first reactor 1.1. An agitator is mounted below the distributor ring. The orifices are spread evenly around the distributor ring. The exit velocity of DMDS through the orifices is 0.63 m/s and the diameter of the orifices is 5 mm.

The temperature in the first reactor 1.1 is about 100 °C. The residence time in the first reactor 1.1, calculated as a quotient of the liquid volume in the first reactor 1.1, divided by the liquid stream which is continuously leaving the first reactor 1.1 through transfer line 8, is about 3.2 h.

The NOₓ-containing offgas stream formed in the first reactor 1.1 and freed from condensable components in the first condenser 5 comprises NO and NO₂ and is passed to the nitric acid regeneration system 39.

The liquid stream which is continuously leaving the first reactor 1.1 is fed to the second reactor 1.2 via the transfer line 8. The temperature in the second reactor 1.2 is about 130° C. The residence time in the second reactor 1.2, calculated as a quotient of the liquid volume in the second reactor 1.2, divided by the liquid stream which is continuously leaving the second reactor 1.2 through transfer line 14, is about 3.5 h.

The NOₓ- containing offgas stream formed in the second reactor 1.2 is freed from condensable components in the second condenser 11. The offgas stream leaving the second condenser 11 comprises NO and NO₂ and the condensate leaving the second condenser 11 comprises nitric acid. Both streams are passed to the nitric acid regeneration system 39.

The liquid stream which is continuously leaving the second reactor 1.2 is passed to the first distillation column 15 via transfer line 14. The first distillation column 15 is operated at a head pressure of from 85 to 100 mbar(abs) and a bottom temperature of 170 to 180° C.

The bottom product of the first distillation column 15, which comprises alkyl sulfonic acid as main component and high boilers like sulfuric acid is withdrawn from the first distillation column 15 as a bottom stream 18 and, after a part of the bottom stream 18 is branched off, fed into a second distillation column 20.

The part of the bottom stream 18 which is branched off, is heated in a heat exchanger 19 and recycled into the bottom part of the first distillation column 15.

The condensate obtained in the condenser 16 with reflux divider contains nitric acid and is fed to the nitric acid regeneration system 39.

In the second distillation column 20, which is operated with a head pressure in the range from 5 to 10 mbar(abs) and a bottom temperature in the range from 180 to 190 °C, the bottom stream of the first distillation column 15 is separated into the top stream 21 comprising low boilers, which is withdrawn at the head of the second distillation column, a bottom stream 22 comprising high boilers, and the mid-boiling fraction 23 comprising pure alkyl sulfonic acid as product stream. The mid-boiling fraction 23 is withdrawn from the second distillation column 20 as a side stream.

The mid-boiling fraction 23 leaving the second distillation column 20 consists of >99,5% strength methanesulfonic acid having a sulfuric acid content of <50 ppm.

The top stream 21 is fed into a condenser 24 with reflux divider, in which the top stream is condensed, and a part of the top stream is recycled into the head section of the second distillation column 20 and the rest of the condensed top stream is withdrawn from the process as light fraction 25. The light fraction 25 leaving the second distillation column 20 consists of water, methanesulfonic acid, methyl methanesulfonate and other low-boiling components.

The bottom stream is divided into a first partial stream which is fed into a heat exchanger 26, in which the first partial stream is heated, and a second partial stream, which is withdrawn from the process as purge stream 27. After heating, the first partial stream is recycled into the second distillation column 20.

The purge stream 27 leaving the second distillation column 20 contains sulfuric acid, methanesulfonic acid and other high-boiling components.

The bottom product leaving the first distillation column 15 contains:

| | |
|---|---|
| methanesulfonic acid: | 97.7 wt% |
| water: | 1.6 wt% |
| sulfuric acid: | 0.3 wt% |

### Example 2:

The reaction was performed in the same set-up as described as in example 1. The process parameters were as described as in example 1, but in this case DMDS was distributed with an exit velocity of DMDS through the nozzles of 1.76 m/s and the nozzle diameter is 3 mm.

The bottom product leaving the first distillation column 15 contains:

| | |
|---|---|
| methanesulfonic acid: | 97.9 wt% |
| water: | 1.6 wt% |
| sulfuric acid: | 0.2 wt% |

A comparison of examples 1 and 2 shows that by reducing the diameter of the nozzles and increasing the exit velocity, the amount of sulfuric acid can be reduced by one third.

### Example 3:

The reaction was performed in experimental set-up 2 which is also according to Fig. 6.

The first reactor 1.1 was charged continuously, while continuously agitated, via the second feed line 3 with pure dimethyl disulfide (>98%) and with 55-65% strength nitric acid in the DMDS (dimethyl disulfide): HNO3 molar ratio of 1 : 5.1.

Dimethyl disulfide is introduced submerged into the liquid phase in the first reactor 1.1 via a distribution ring.

The distribution ring has 1.2 orifices per cubic meter of reaction volume in the first reactor 1.1. An agitator is mounted below the distributor ring. The orifices are spread evenly around the distributor ring. The exit velocity of DMDS through the orifices is 2.51 m/s and the diameter of the orifices is 3 mm.

The temperature in the first reactor 1.1 is about 100 °C. The residence time in the first reactor 1.1, calculated as a quotient of the liquid volume in the first reactor 1.1, divided by the liquid stream which is continuously leaving the first reactor 1.1 through transfer line 8, is about 2.7 h.

The NOₓ-containing offgas stream formed in the first reactor 1.1 and freed from condensable components in the first condenser 11 comprises NO and NO₂ and is passed to the nitric acid regeneration system 39.

The liquid stream which is continuously leaving the first reactor 1.1 is fed to the second reactor 1.2 via the transfer line 8. The temperature in the second reactor 1.2 is about 130 °C. The residence time in the second reactor 1.2, calculated as a quotient of the liquid volume in the second reactor 1.2, divided by the liquid stream which is continuously leaving the second reactor 1.2 through transfer line 14, is about 3.2 h.

The NOₓ- containing offgas stream formed in the second reactor 1.2 is freed from condensable components in the second condenser 11. The offgas stream leaving the second condenser 11 comprises NO and NO₂ and the condensate leaving the second condenser 11 comprises nitric acid. Both streams are passed to the nitric acid regeneration system 39.

The liquid stream which is continuously leaving the second reactor 1.2 is passed to the first distillation column 15. The first distillation column 15 is operated at a head pressure of from 85 to 100 mbar(abs) and a bottom temperature of 170 to 180 °C. The bottom product leaving the first distillation column 15 is transferred to the second distillation column 20.

The condensate obtained in the third condenser 16 with reflux divider contains nitric acid and is fed to the nitric acid regeneration system 39.

The mid-boiling fraction 23 leaving the second distillation column 20 as side stream consists of >99,5% strength methanesulfonic acid having a sulfuric acid content of <50 ppm. The light fraction 25 leaving the second distillation column 20 consists of water, methanesulfonic acid, methyl methanesulfonate and other low-boiling components.

The purge stream 27 leaving the second distillation column 20 consists of sulfuric acid, methanesulfonic acid and other high-boiling components.

The bottom product leaving the first distillation column 15 contains:

| | |
|---|---|
| methanesulfonic acid: | 97.0 wt% |
| water: | 2.0 wt% |
| sulfuric acid: | 0.3 wt% |

### Example 4:

The reaction was performed in the same set-up as described as in example 3. The process parameters were as described as in example 3, but in this case DMDS was distributed with a distributor ring with 1.7 nozzles per cubic meter of reaction volume and an exit velocity of DMDS through the nozzles of 7.45 m/s and the diameter of the orifices is 1.5 mm.

The bottom product leaving the first distillation column 15 contains:

| | |
|---|---|
| methanesulfonic acid: | 97.2 wt% |
| water: | 2.0 wt% |
| sulfuric acid: | 0.2 wt% |

By the comparison of the composition of the bottom product leaving the first distillation column 15 of examples 3 and 4, it can be seen that by reducing the diameter of the orifices and increasing the exit velocity of the DMDS, as well as increasing the amount of orifices per reaction volume, the amount of sulfuric acid can be reduced further.

### Example 5:

The reaction was performed in the same set-up as described in example 3. However, in this case the exit velocity of DMDS through the nozzles is 1.27 m/s. The residence time in the first reactor 1.1 was increased from 2.7 h to 5.2 h and in the second reactor 1.2 from 3.2 h to 6.3 h. All other process parameters were as described in example 3.

The bottom product leaving the first distillation column 15 contains:

| | |
|---|---|
| methanesulfonic acid: | 97.1 wt% |
| water: | 2.0 wt% |
| sulfuric acid: | 0.3 wt% |

The comparison with the results from example 3 shows that a reduced exit velocity of DMDS of 1,27 m/s and a longer residence time in the first reactor 1.1 and the second reactor 1.2 has no effect on formation of sulfuric acid.

### Comparative Example:

The reaction was performed in the same set-up as described in example 1. The process parameters were as described as in example 1, but in this case DMDS was distributed with an exit velocity of DMDS through the nozzles of 0.41 m/s and the nozzle diameter is 5 mm.

The residence time in the first reactor 1.1 was increased from about 3.2 h to about 4.5 h and in the second reactor 1.2 from about 3.5 h to about 5.4 h. All other process parameters were as described in example 1.

The bottom product leaving the first distillation column 15 contains:

| | |
|---|---|
| methanesulfonic acid: | 97.7 wt% |
| water: | 1.7 wt% |
| sulfuric acid: | 0.4 wt% |

A comparison of example 1 and the comparative example shows that by reducing the exit velocity through the orifices below 0,63 m/s the amount of sulfuric acid that is produced increases significantly.

### List of reference numbers

- 1: reactor
- 1.1: first reactor
- 1.2: second reactor
- 2: first feed line
- 3: second feed line
- 4: gas line
- 5: first condenser
- 6: recycle line
- 7: gas line
- 8: transfer line
- 10: gas line
- 11: second condenser
- 12: feed line
- 13: gas line
- 14: transfer line
- 15: first distillation column
- 16: third condenser
- 17: reflux
- 18: line
- 19: first reboiler
- 20: second distillation column
- 21: top stream
- 22: bottom stream
- 23: mid-boiler fraction
- 24: forth condenser
- 25: light fraction
- 26: second reboiler
- 27: purge stream
- 28: orifices at the bottom of the reactor
- 29: orifices at the wall of the reactor
- 30: mixing device
- 31: dip tube
- 32: distributor
- 33: orifice
- 34: external fluid circulation
- 35: pump
- 36: ejector nozzle
- 37: nozzle
- 38: static mixer
- 39: regeneration for nitric acid
- 40: distillation
- 41: reaction apparatus
- 43: air
- 44: water
- 45: nitric acid
- 46: inner tube
- 47: first end
- 48: second end

## Claims

1. A process for producing alkyl sulfonic acid in a reaction apparatus (41) which contains a liquid phase comprising aqueous nitric acid, wherein dialkyl disulfide is fed submerged into the liquid phase in the reaction apparatus (41) and a crude reaction product is formed in the reaction apparatus (41) by chemical reaction of the dialkyl disulfide with the nitric acid, wherein feeding the dialkyl disulfide comprises at least one of:
- the liquid dialkyl disulfide is fed into the reaction apparatus (41) through an orifice (28, 29; 33) having a hydraulic diameter of less than 5 mm;
- the liquid dialkyl disulfide is fed into the reaction apparatus (41) with an inlet velocity of at least 0.6 m/s.

2. The process according to claim 1, wherein the reaction apparatus comprises a first reactor (1.1) into which the liquid dialkyl disulfide and the nitric acid are fed and in which a first crude reaction product is formed, and a second reactor (1.2) into which the first crude reaction product is fed and in which the reaction for forming the alkyl sulfonic acid is completed, thereby forming the crude reaction product.

3. The process according to claim 1 or 2, wherein the crude reaction product is worked-up to obtain a pure alkyl sulfonic acid.

4. The process according to claim 3, wherein working-up the crude reaction product comprises at least one distillation step.

5. The process according to any of claims 1 to 4, wherein the reaction apparatus comprises a reactor (1; 1.1, 1.2) with an external fluid circulation (34).

6. The process according to claim 5, wherein the dialkyl disulfide is fed into the external fluid circulation (34).

7. The process according to any of claims 1 to 4, wherein the reaction apparatus (41) comprises a jet loop reactor.

8. The process according to any of claims 1 to 6, wherein the reaction apparatus (41) comprises a tank reactor.

9. The process according to claim 8, wherein the dialkyl disulfide is fed into the tank reactor through a plurality of orifices (28, 29) at the bottom and/or the walls of the tank reactor, through a dip tube having at least one opening, or through a distributor (32) having a plurality of orifices (33).

10. The process according to any of claims 1 to 9, wherein the liquid dialkyl disulfide is fed into the reactor through a plurality of orifices (28,29; 33), wherein the number of orifices is larger than 1.0 orifices per cubic meter reaction volume.

11. The process according to any of claims 1 to 10, wherein the reactor (1) comprises an additional mixing device (30).

12. The process according to any of claims 1 to 11, wherein the dialkyl disulfide is dimethyl disulfide or diethyl disulfide and the alkyl sulfonic acid is methyl sulfonic acid or ethyl sulfonic acid.

## Patentansprüche

1. Verfahren zur Herstellung von Alkylsulfonsäure in einer Reaktionsvorrichtung (41), die eine flüssige Phase enthält, die wässrige Salpetersäure umfasst, wobei Dialkyldisulfid untergetaucht in die flüssige Phase in der Reaktionsvorrichtung (41) eingespeist wird und ein rohes Reaktionsprodukt in der Reaktionsvorrichtung (41) durch chemische Reaktion des Dialkyldisulfids mit der Salpetersäure gebildet wird, wobei das Einspeisen des Dialkyldisulfids mindestens eines der folgenden umfasst:
- das flüssige Dialkyldisulfid wird in die Reaktionsvorrichtung (41) durch eine Öffnung (28, 29; 33) mit einem hydraulischen Durchmesser von weniger als 5 mm eingespeist;
- das flüssige Dialkyldisulfid wird in die Reaktionsvorrichtung (41) mit einer Einströmgeschwindigkeit von mindestens 0,6 m/s eingespeist.

2. Verfahren nach Anspruch 1, wobei die Reaktionsvorrichtung einen ersten Reaktor (1.1), in den das flüssige Dialkyldisulfid und die Salpetersäure eingespeist werden und in dem ein erstes rohes Reaktionsprodukt gebildet wird, und einen zweiten Reaktor (1.2) umfasst, in den das erste rohe Reaktionsprodukt eingespeist wird und in dem die Reaktion zur Bildung der Alkylsulfonsäure abgeschlossen wird, wodurch das rohe Reaktionsprodukt gebildet wird.

3. Verfahren nach Anspruch 1 oder 2, wobei das rohe Reaktionsprodukt aufgearbeitet wird, um eine reine Alkylsulfonsäure zu erhalten.

4. Verfahren nach Anspruch 3, wobei die Aufarbeitung des rohen Reaktionsprodukts mindestens einen Destillationsschritt umfasst.

5. Verfahren nach einem der Ansprüche 1 bis 4, wobei die Reaktionsvorrichtung einen Reaktor (1; 1.1, 1.2) mit einem externen Fluidkreislauf (34) umfasst.

6. Verfahren nach Anspruch 5, wobei das Dialkyldisulfid in den externen Fluidkreislauf (34) eingespeist wird.

7. Verfahren nach einem der Ansprüche 1 bis 4, wobei die Reaktionsvorrichtung (41) einen Strahlschlaufenreaktor umfasst.

8. Verfahren nach einem der Ansprüche 1 bis 6, wobei die Reaktionsvorrichtung (41) einen Tankreaktor umfasst.

9. Verfahren nach Anspruch 8, wobei das Dialkyldisulfid in den Tankreaktor durch eine Vielzahl von Öffnungen (28, 29) am Boden und/oder den Wänden des Tankreaktors, durch ein Tauchrohr mit mindestens einer Öffnung oder durch einen Verteiler (32) mit einer Vielzahl von Öffnungen (33) eingespeist wird.

10. Verfahren nach einem der Ansprüche 1 bis 9, wobei das flüssige Dialkyldisulfid durch eine Vielzahl von Öffnungen (28, 29; 33) in den Reaktor eingespeist wird, wobei die Anzahl der Öffnungen größer als 1,0 Öffnungen pro Kubikmeter Reaktionsvolumen ist.

11. Verfahren nach einem der Ansprüche 1 bis 10, wobei der Reaktor (1) eine zusätzliche Mischvorrichtung (30) umfasst.

12. Verfahren nach einem der Ansprüche 1 bis 11, wobei das Dialkyldisulfid Dimethyldisulfid oder Diethyldisulfid ist und die Alkylsulfonsäure Methansulfonsäure oder Ethansulfonsäure ist.

## Revendications

1. Un procédé de production d'acide alkylsulfonique dans un appareil de réaction (41) qui contient une phase liquide comprenant de l'acide nitrique aqueux, dans lequel du dialkyle disulfure est introduit en immersion dans la phase liquide dans l'appareil de réaction (41) et un produit de réaction brut est formé dans l'appareil de réaction (41) par réaction chimique du dialkyle disulfure avec l'acide nitrique, dans lequel l'introduction du dialkyle disulfure comprend au moins l'une des opérations suivantes :
- le dialkyle disulfure liquide est introduit dans l'appareil de réaction (41) à travers un orifice (28, 29 ; 33) ayant un diamètre hydraulique inférieur à 5 mm ;
- le dialkyle disulfure liquide est introduit dans l'appareil de réaction (41) avec une vitesse d'entrée de au moins 0,6 m/s.

2. Le procédé selon la revendication 1, dans lequel l'appareil de réaction comprend un premier réacteur (1.1) dans lequel le dialkyle disulfure liquide et l'acide nitrique sont introduits et dans lequel un premier produit de réaction brut est formé, et un second réacteur (1.2) dans lequel le premier produit de réaction brut est introduit et dans lequel la réaction de formation de l'acide alkylsulfonique est achevée, formant ainsi le produit de réaction brut.

3. Le procédé selon la revendication 1 ou 2, dans lequel le produit de réaction brut est traité pour obtenir un acide alkylsulfonique pur.

4. Le procédé selon la revendication 3, dans lequel le traitement du produit de réaction brut comprend au moins une étape de distillation.

5. Le procédé selon l'une quelconque des revendications 1 à 4, dans lequel l'appareil de réaction comprend un réacteur (1 ; 1.1, 1.2) avec une circulation de fluide externe (34).

6. Le procédé selon la revendication 5, dans lequel le dialkyle disulfure est introduit dans la circulation de fluide externe (34).

7. Le procédé selon l'une quelconque des revendications 1 à 4, dans lequel l'appareil de réaction (41) comprend un réacteur à boucle à jet.

8. Le procédé selon l'une quelconque des revendications 1 à 6, dans lequel l'appareil de réaction (41) comprend un réacteur à cuve.

9. Le procédé selon la revendication 8, dans lequel le dialkyle disulfure est introduit dans le réacteur à cuve à travers une pluralité d'orifices (28, 29) au fond et/ou sur les parois du réacteur à cuve, à travers un tube plongeur ayant au moins une ouverture, ou à travers un distributeur (32) ayant une pluralité d'orifices (33).

10. Le procédé selon l'une quelconque des revendications 1 à 9, dans lequel le dialkyle disulfure liquide est introduit dans le réacteur à travers une pluralité d'orifices (28, 29 ; 33), dans lequel le nombre d'orifices est supérieur à 1,0 orifice par mètre cube de volume de réaction.

11. Le procédé selon l'une quelconque des revendications 1 à 10, dans lequel le réacteur (1) comprend un dispositif de mélange supplémentaire (30).

12. Le procédé selon l'une quelconque des revendications 1 à 11, dans lequel le dialkyle disulfure est le diméthyle disulfure ou le diéthyle disulfure et l'acide alkylsulfonique est l'acide méthylsulfonique ou l'acide éthylsulfonique.
